# DEMANDE DE BREVET EUROPEEN

(11) **EP 1 535 899 A1**
(43) Date de publication de la demande: **01.06.2005**
(21) Numéro de dépôt: 04292647.7
(22) Date de dépôt: 09.11.2004
(51) Int. Cl.: C07C 211/53, C07C 215/14, C07C 217/08, C07C 215/16

(54) **Composition tinctoriale comprenant au moins une p-phénylènediamine tertiaire particulière, procédé de coloration la mettant en oeuvre**

(30) Priorité: 28.11.2003 FR 0314021
(71) Demandeur: L'OREAL, 75008 Paris (FR)
(72) Inventeur: Metais, Eric, 95320 St. Leu la Fôret (FR); Radisson, Xavier, 75014 Paris (FR); Sabelle, Stéphane, 75005 Paris (FR)
(74) Mandataire: Fevrier, Murielle

(57) **Abrégé**

La présente invention a pour un composé de formule suivente (I) 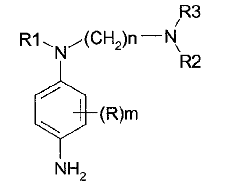 ainsi que ses sels d'addition,
dans laquelle R représente un radical alkyle, linéaire ou ramifié, en C₁-C₄ ; un radical alcoxy, linéaire ou ramifié, en C₁-C₄ ; m représente un entier compris entre 0 et 4 ; R₁ représente un radical alkyle, linéaire ou ramifié, en C₁-C₄ ; un radical hydroxyalkyle, linéaire ou ramifié, en C₂-C₄ ; un radical alcoxy(C₁-C₄) alkyle en C₂-C₄, linéaire ou ramifié ; R₂, représente un atome d'hydrogène ; un radical alkyle, linéaire ou ramifié, en C₁-C₄ ; un radical hydroxyalkyle, linéaire ou ramifié, en C₂-C₄ ; un radical alcoxy(C₁-C₄) alkyle en C₂-C₄, linéaire ou ramifié ; n représente un entier compris entre 2 et 6.

Elle a de plus pour objet une composition tinctoriale comprenant ledit composé, un procédé de coloration de fibres kératiniques, notamment humaines, mettant en jeu une telle composition, ainsi qu'un dispositif à compartiments adapté.

## Description

L'invention a pour objet une composition tinctoriale comprenant une para-phénylène diamine particulière, ainsi qu'un procédé de coloration de fibres kératiniques mettant en oeuvre ladite composition. Elle a de même pour objet des para-phénylène diamines particulières en tant que telles ainsi qu'une composition tinctoriale les comprenant.

Il est connu de teindre les fibres kératiniques et en particulier les cheveux humains avec des compositions tinctoriales contenant des précurseurs de colorant d'oxydation, appelés généralement bases d'oxydation. Ces bases d'oxydation sont des composés incolores ou faiblement colorés qui, associés à des produits oxydants, peuvent donner naissance par un processus de condensation oxydative à des composés colorés. On sait également que l'on peut faire varier les nuances obtenues en associant ces bases d'oxydation à des coupleurs ou modificateurs de coloration.

La variété des molécules mises en jeu au niveau des bases d'oxydation et des coupleurs, permet l'obtention d'une riche palette de couleurs.

Ce procédé de coloration d'oxydation consiste à appliquer sur les fibres kératiniques, des bases ou un mélange de bases et de coupleurs avec de l'eau oxygénée à titre d'agent oxydant, à laisser pauser, puis à rincer les fibres. Généralement appliquées à pH basique, il permet d'obtenir une teinture et simultanément un éclaircissement de la fibre qui se traduit en pratique par la possibilité d'obtenir une coloration finale plus claire que la couleur d'origine.

Ce type de coloration doit par ailleurs satisfaire un certain nombre d'exigences. Ainsi, il doit être sans inconvénient sur le plan toxicologique, doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine et la para-toluènediamine sont des bases d'oxydation largement utilisées. Elles permettent d'obtenir avec des coupleurs d'oxydation des nuances variées.

Cependant, il existe un besoin de découvrir de nouvelles bases d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de la ténacité aux agents extérieurs.

Ce but est atteint avec la présente invention qui a pour premier objet un composé de formule (I) suivante : 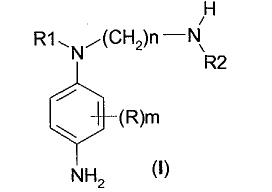 ainsi que ses sels d'addition,
dans laquelle :
- R représente un radical alkyle, linéaire ou ramifié, en C₁-C₄ ; un radical alcoxy, linéaire ou ramifié, en C₁-C₄.
- m représente un entier compris entre 0 et 4 ;
- R₁ représente un radical alkyle, linéaire ou ramifié, en C₁-C₄ ; un radical hydroxyalkyle, linéaire ou ramifié, en C₂-C₄ un radical alcoxy(C₁-C₄) alkyle en C₂-C₄, linéaire ou ramifié ;
- R₂, représente un atome d'hydrogène ; un radical alkyle, linéaire ou ramifié, en C₁-C₄ ; un radical hydroxyalkyle, linéaire ou ramifié, en C₂-C₄ ; un radical alcoxy(C₁-C₄) alkyle en C₂-C₄, linéaire ou ramifié ;
- n représente un entier compris entre 2 et 6.

Elle a de même pour objet une composition tinctoriale comprenant dans un milieu approprié pour la teinture des fibres kératiniques, en particulier humaines, au moins un composé de formule (I) précitée.

Un autre objet de l'invention est constitué par un procédé de coloration des fibres kératiniques, notamment humaines, mettant en oeuvre ladite composition.

Elle concerne de plus un dispositif à plusieurs compartiments pour la mise en oeuvre dudit procédé.

D'autres caractéristiques et avantages de la présente invention apparaîtront plus clairement à la lecture de la description et de l'exemple qui vont suivre.

La composé de formule (I) va tout d'abord être décrit.

Conformément à un mode de réalisation particulier de l'invention, le ou les radicaux R, identiques ou non, représentent un radical alkyle en C₁-C₂. De préférence, le ou les radicaux R représentent, chacun, un radical méthyle.

De préférence, dans la formule (I), m est compris entre 0 et 2, plus particulièrement, m est égal à 0 ou 1.

En ce qui concerne R₁, celui-ci représente plus particulièrement dans la formule (I) un radical alkyle en C₁-C₂ ; un radical hydroxyalkyle en C₂ ; un radical alcoxy(C₁-C₄) alkyle en C₂. De préférence, R₁ représente un radical méthyle, éthyle, hydroxyéthyle.

Pour ce qui a trait à R₂, ce radical représente plus spécialement un atome d'hydrogène, un radical alkyle en C₁- C₂ ; un radical hydroxyalkyle en C₂ ; un radical alcoxy(C₁-C₄) alkyle en C₂.

De préférence, R₂ représente un atome d'hydrogène, un radical méthyle, un radical, hydroxyéthyle.

Conformément à un mode de réalisation de l'invention, le composé de formule (I) est choisi parmi les composés suivants, ainsi que leurs sels d'addition :

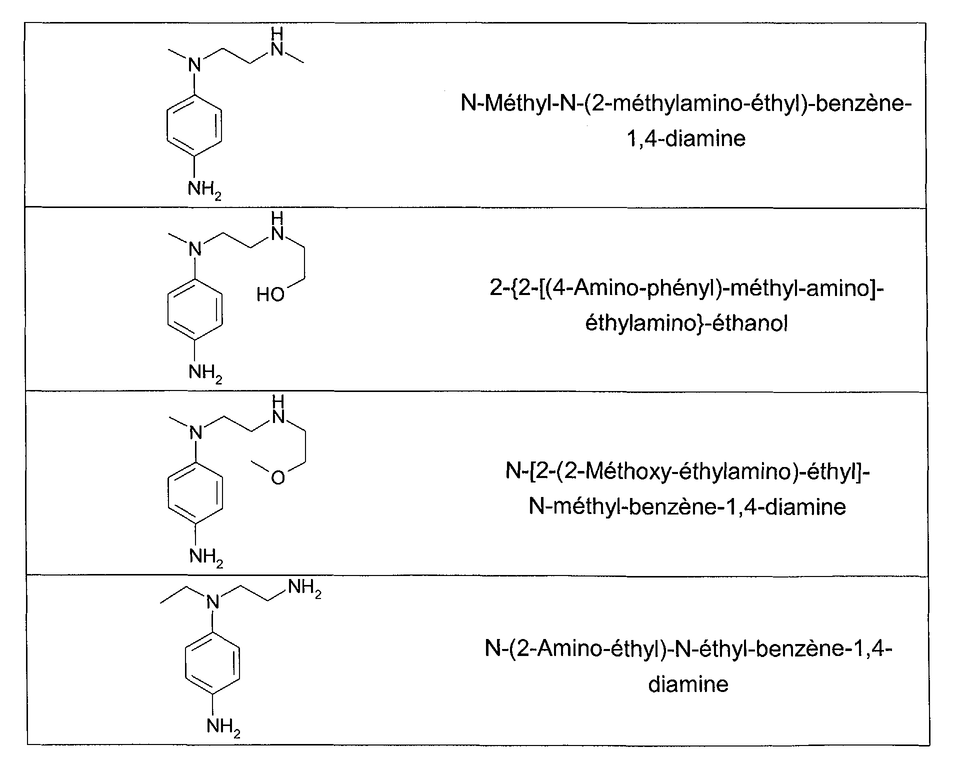

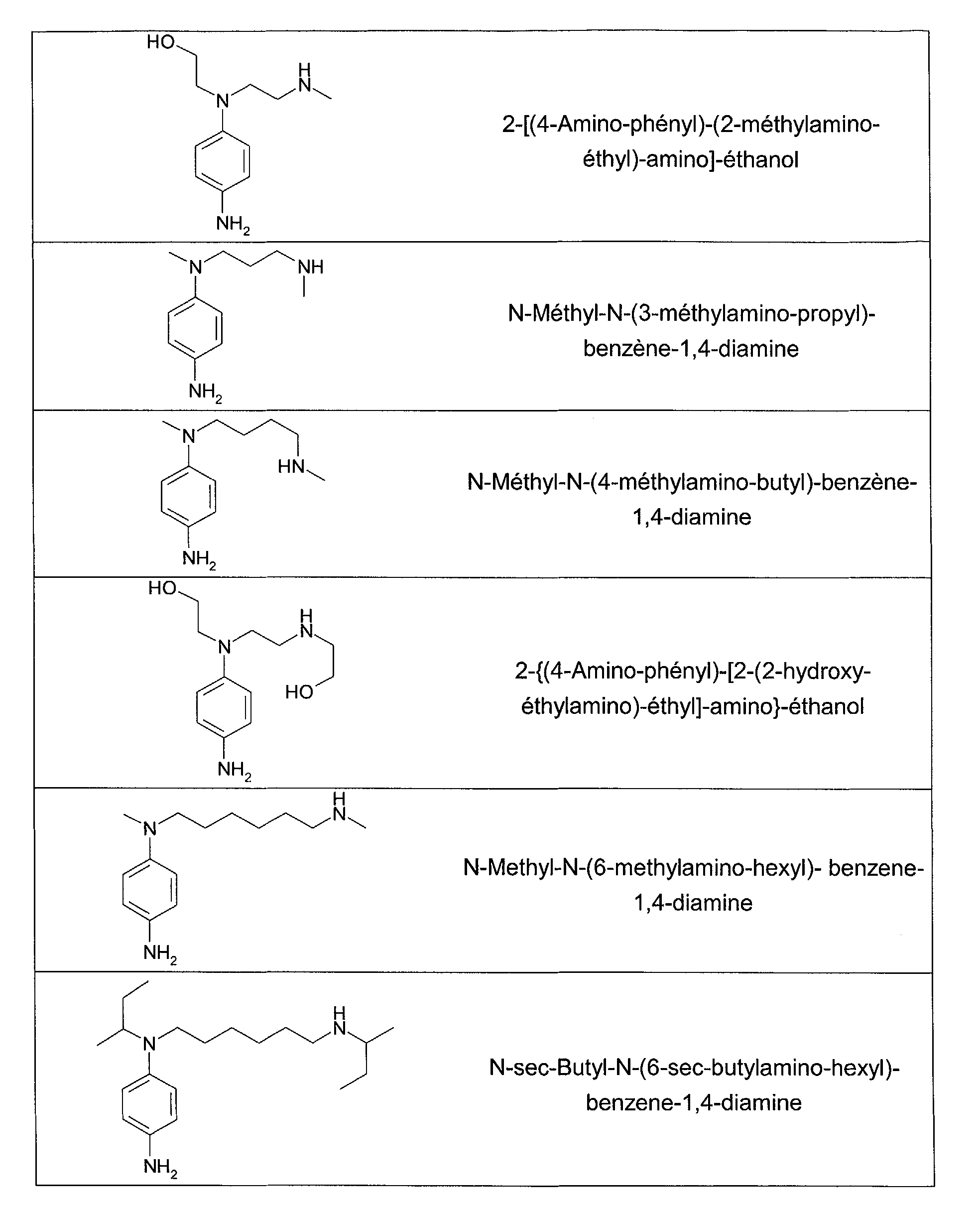

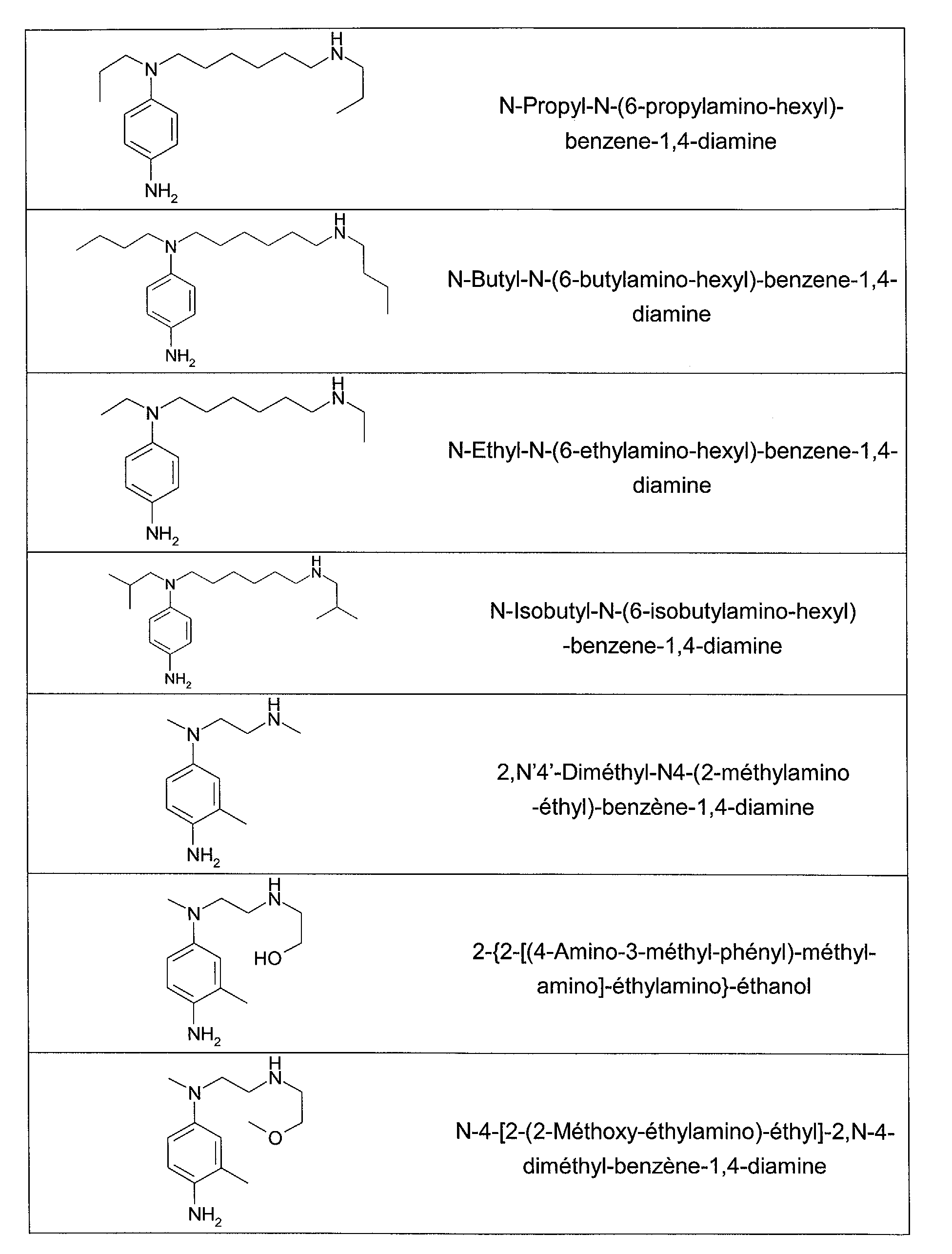

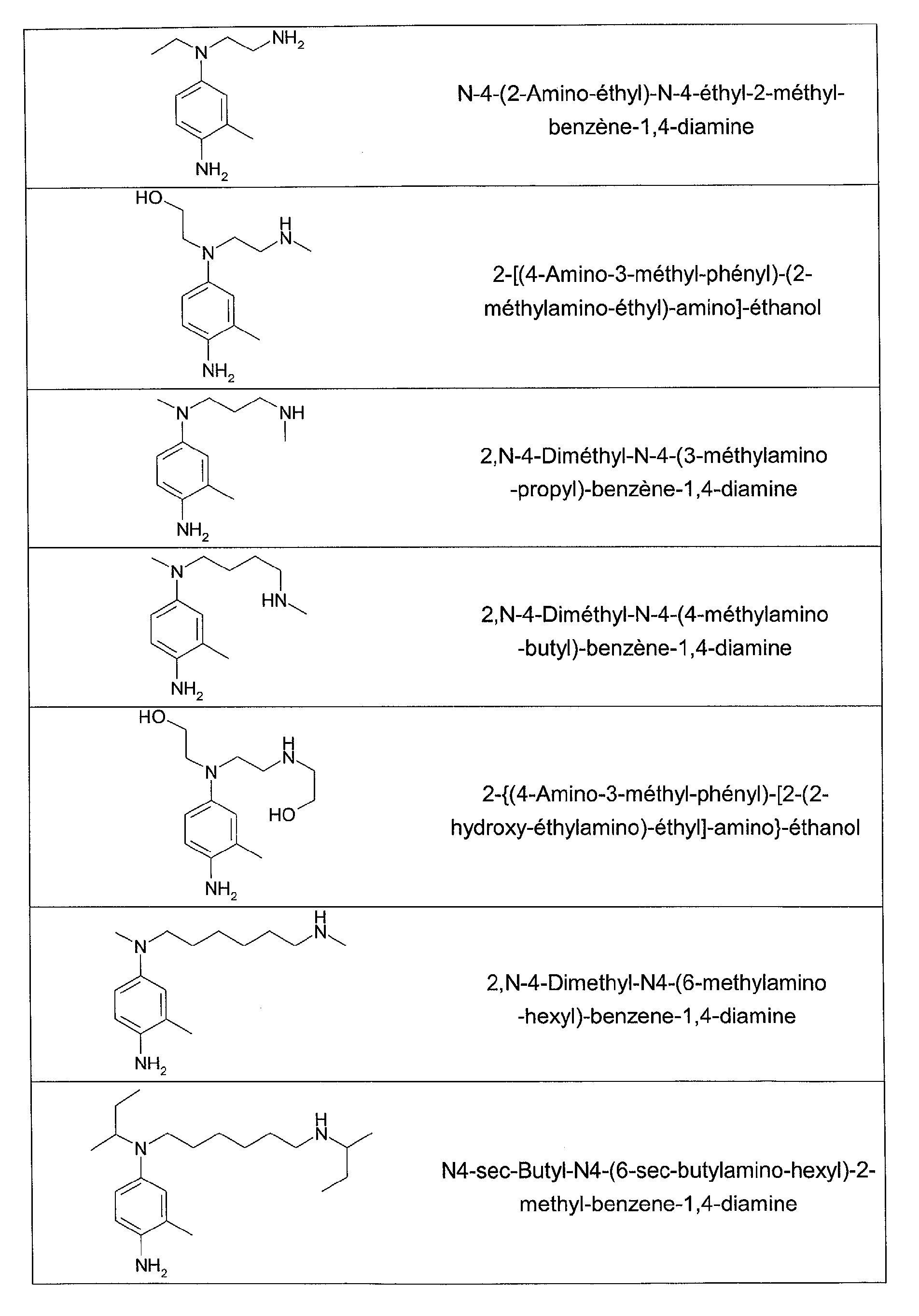

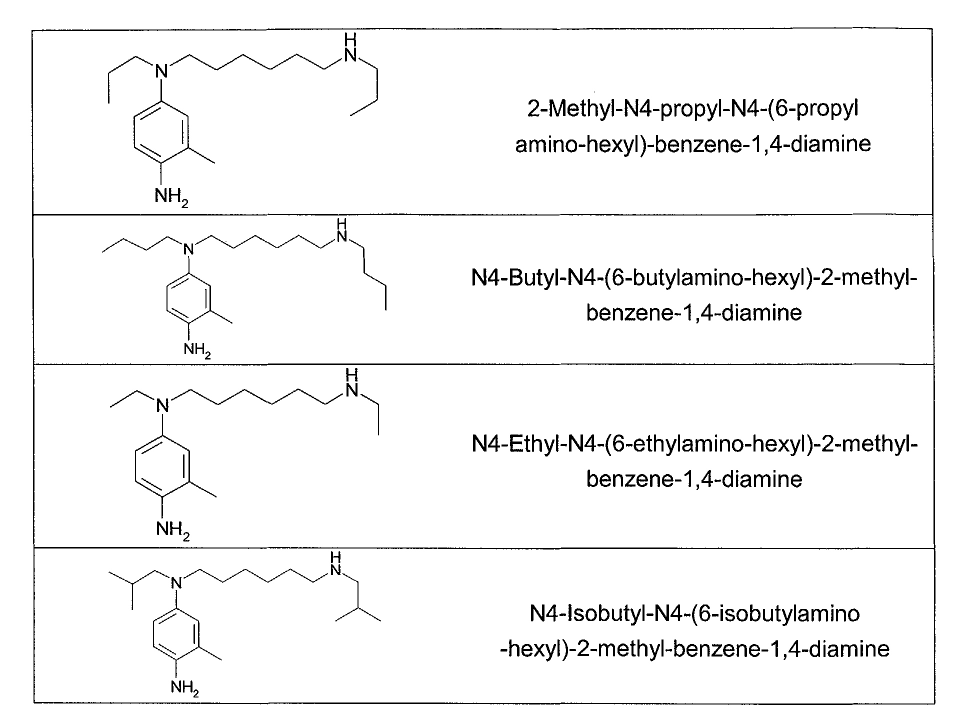

D'une manière générale, les sels d'addition du composé de formule (I) sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Les composés de formules (I) peuvent être synthétisés selon le schéma réactionnel suivant :

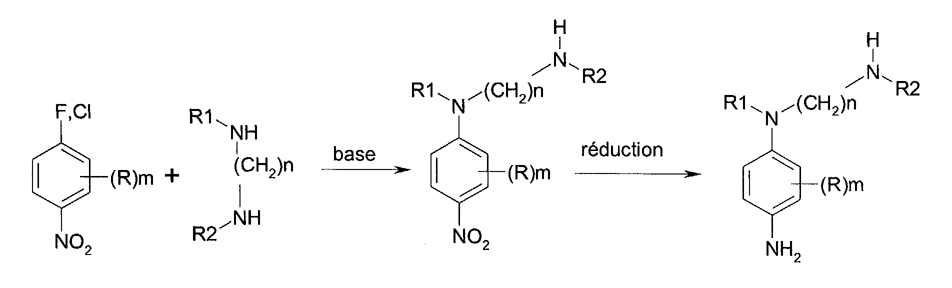

La première étape s'inspire des synthèses décrites dans "Synthesis", 1990 (12), 1147-148 et "Synth Commun" 1990, 20 (22), 3537-3545, auxquelles on pourra se référer.

La deuxième étape est une étape de réduction classique notamment décrite par exemple dans la demande de brevet DE 42 41 532, ainsi que dans "J. Heterocycl. Chem." 1992, 29 (6) 1513-1517, auxquels on pourra également se reporter.

Un autre objet de la présente invention est donc constitué par une composition tinctoriale comprenant, dans un milieu approprié pour la teinture de fibres kératiniques, notamment humaines, au moins un composé de formule (I) tel que décrit précédemment.

Plus particulièrement, ledit composé est présent en tant que base d'oxydation.

Conformément à un mode de réalisation avantageux de l'invention, la teneur en composé de formule (I) comprise entre 0,001 et 10 % en poids par rapport au poids de la composition tinctoriale, de préférence entre 0,005 et 6 % en poids par rapport au poids de la composition tinctoriale.

La composition tinctoriale selon l'invention peut aussi comprendre une ou plusieurs bases d'oxydation additionnelles éventuellement associée à au moins un coupleur, choisis parmi ceux conventionnellement utilisées pour la teinture de fibres kératiniques.

A titre d'exemple, ces bases d'oxydation additionnelles sont choisies parmi les para-phénylènediamines différentes de celles du composé de formule (I), les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles, lorsqu'elles sont présentes dans la composition de l'invention, sont en général présentes chacune en quantité comprise entre 0,001 à 10 % en poids environ par rapport au poids de la composition tinctoriale, de préférence entre 0,005 et 6 % en poids par rapport au poids de la composition tinctoriale.

Comme indiqué auparavant, la composition tinctoriale de l'invention comprend au moins un coupleur.

Parmi ces coupleurs, on peut notamment citer les méta-phénylènediamines, les méta-aminophénols, les méta-diphénols, les coupleurs naphtaléniques, les coupleurs hétérocycliques, tels que par exemple les dérivés indoliques, les dérivés indoliniques, le sésamol et ses dérivés, les dérivés pyridiniques, les dérivés pyrazolotriazoles, les pyrazolones, et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(ß-hydroxyéthylamino)toluène et leurs sels d'addition avec un acide.

Dans la composition de la présente invention, le ou les coupleurs sont chacun généralement présents en quantité comprise entre 0,001 et 10 % en poids environ par rapport au poids de la composition tinctoriale, de préférence entre 0,005 et 6 % en poids environ par rapport au poids de la composition tinctoriale.

Comme précédemment, les sels d'addition des bases d'oxydation additionnelles et des coupleurs sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La composition selon l'invention comprend de même éventuellement au moins un colorant direct, qui est avantageusement choisi parmi des espèces anioniques, cationiques ou non ioniques.

A titre d'exemples, on peut citer les colorants benzéniques nitrés, les colorants azoïques, azométhiniques, aziniques, méthiniques, tétraazapenthaméthiniques, quinoniques, anthraquinoniques, naphtoquinoniques, benzoquinoniques, phénotiaziniques, indigoïdes, indoaminiques, xanthéniques, phénanthridiniques, phtalocyanines, ceux dérivés du triarylméthane, les colorants naturels, seuls ou en mélanges.

Parmi les chromophores nitro utilisables selon l'invention, on peut citer de manière non limitative les radicaux issus des composés suivants:
-1,4-diamino-2-nitrobenzène
-1-amino-2 nitro-4-β- hydroxyéthylaminobenzène
-1-amino-2 nitro-4-bis(β-hydroxyéthyl)-aminobenzène
-1,4-bis(β-hydroxyéthylamino)-2-nitrobenzène
-1-β-hydroxyéthylamino-2-nitro-4-bis-(β-hydroxyéthylamino)-benzène
- 1-β-hydroxyéthylamino-2-nitro-4-aminobenzène
-1-β-hydroxyéthylamino-2-nitro-4-(éthyl)(β-hydroxyéthyl)-aminobenzène
-1-amino-3-méthyl-4-β-hydroxyéthylamino-6-nitrobenzène
-1-amino-2-nitro-4-β-hydroxyéthylamino-5-chlorobenzène
-1,2-diamino-4-nitrobenzène
-1-amino-2-β-hydroxyéthylamino-5-nitrobenzène
-1,2-bis-(β-hydroxyéthylamino)-4-nitrobenzène
-1-amino-2-tris-(hydroxyméthyl)-méthylamino-5-nitrobenzène
-1-hydroxy-2-amino-5-nitrobenzène
-1-hydroxy-2-amino-4-nitrobenzène
-1-hydroxy-3-nitro-4-aminobenzène
-1-hydroxy-2-amino-4,6-dinitrobenzène
-1-β-hydroxyéthyloxy-2-β-hydroxyéthylamino-5-nitrobenzène
-1-méthoxy-2-β-hydroxyéthylamino-5-nitrobenzène
-1-β-hydroxyéthyloxy-3-méthylamino-4-nitrobenzène
-1-β,γ-dihydroxypropyloxy-3-méthylamino-4-nitrobenzène
-1-β-hydroxyéthylamino-4-β,γ-dihydroxypropyloxy-2-nitrobenzène
-1-β,γ-dihydroxypropylamino-4-trifluorométhyl-2-nitrobenzène
-1-β-hydroxyéthylamino-4-trifluorométhyl-2-nitrobenzène
-1-β-hydroxyéthylamino-3-méthyl-2-nitrobenzène
-1-β-aminoéthylamino-5-méthoxy-2-nitrobenzène
-1-hydroxy-2-chloro-6-éthylamino-4-nitrobenzène
-1-hydroxy-2-chloro-6-amino-4-nitrobenzène
-1-hydroxy-6-bis-(β-hydroxyéthyl)-amino-3-nitrobenzène
-1-β-hydroxyéthylamino-2-nitrobenzène
-1-hydroxy-4-β-hydroxyéthylamino-3-nitrobenzène.

Parmi les colorants directs azoïques, on peut citer ceux décrits dans les demandes de brevets WO 95/15144, WO-95/01772 et EP-714954. On peut également citer parmi les colorants azoïques suivants, décrits dans le COLOUR INDEX INTERNATIONAL 3^{e} édition, comme les Disperse Red 17 ; Acid Yellow 9 ; Acid Black 1 ; Basic Red 22 ; Basic Red 76 ; Basic Yellow 57 ; Basic Brown 16 ; Acid Yellow 36 ; Acid Orange 7 ; Acid Red 33 ; Acid Red 35 ; Basic Brown 17 ; Acid Yellow 23 ; Acid Orange 24 ; Disperse Black 9. On peut aussi citer le 1-(4'-aminodiphénylazo)-2-méthyl-4bis-(β-hydroxyéthyl) aminobenzène et l'acide 4-hydroxy-3-(2-méthoxyphénytazo)-1-naphtalène sulfonique.

Parmi les colorants quinoniques, on peut citer les radicaux issus des colorants suivants : Disperse Red 15 ; Solvent Violet 13 ; Acid Violet 43 ; Disperse Violet 1 ; Disperse Violet 4 ; Disperse Blue 1 ; Disperse Violet 8 ; Disperse Blue 3 ; Disperse Red 11 ; Acid Blue 62 ; Disperse Blue 7 ; Basic Blue 22 ; Disperse Violet 15 ; Basic Blue 99 ; ainsi que les composés suivants : -1-N-méthylmorpholiniumpropylamino-4-hydroxyanthraquinone ; 1-aminopropylamino-4-méthylaminoanthraquinone ; 1-aminopropylaminoanthraquinone ; 5-β-hydroxyéthyl-1,4-diaminoanthraquinone ; 2-aminoéthylaminoanthraquinone ; 1,4-bis-(β,γ-dihydroxypropylamino)-anthraquinone.

Parmi les colorants directs aziniques, on peut citer les composés suivants : Basic Blue 17 ; Basic Red 2.

Parmi les colorants directs indoaminiques, on peut citer les composés suivants : 2-β-hydroxyéthlyamino-5-[bis-(β-4'-hydroxyéthyl)amino]anilino-1,4-benzoquinone ; 2-β-hydroxyéthylamino-5-(2'-méthoxy-4'-amino)anilino-1,4-benzoquinone ; 3-N(2'-Chloro-4'-hydroxy)phényl-acétylamino-6-méthoxy-1,4-benzoquinone imine ; 3-N(3'-Chloro-4'-méthylamino)phényl-uréido-6-méthyl-1,4-benzoquinone imine ; 3-[4'-N-(éthyl, carbamyl méthyl)-amino]-phényl-uréido-6-méthyl-1,4-benzoquinone imine.

On peut aussi citer les colorants décrits dans les documents US 5888252, EP 1133975, WO 03/029359, EP 860636, WO 95/01772, WO 95/15144, EP 714954. On peut aussi citer ceux cités dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitres de l'encyclopédie "ULLMANN's ENCYCLOPEDIA of Industrial chemistry" 7th édition, Wiley and sons.

S'ils sont présents, la teneur en colorant(s) direct(s) dans la composition varie en général de 0,001 à 20% en poids, par rapport au poids de la composition tinctoriale, et de préférence de 0,01 à 10% en poids, par rapport au poids de la composition tinctoriale.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions comprises entre 1 et 40 % en poids environ par rapport au poids de la composition tinctoriale, et encore plus préférentiellement entre 5 et 30 % en poids environ.

La composition de l'invention peut aussi contenir au moins un agent oxydant.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 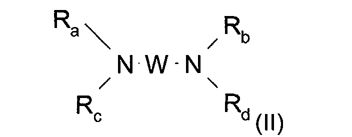 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres kératiniques, sèches ou humides, la composition selon l'invention, telle que définie précédemment.

Il est à noter que l'agent oxydant peut être ajouté à la composition tinctoriale, au moment de l'emploi ou bien il peut encore être mis en oeuvre à partir d'une composition oxydante le contenant, appliquée simultanément ou séquentiellement à la composition tinctoriale selon l'invention. Dans ce dernier cas, l'agent oxydant est contenu dans une composition différente de ladite composition tinctoriale selon l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour obtenir la coloration souhaitée.

Le mélange obtenu est ensuite appliqué sur les fibres kératiniques. Après un temps de pose de 3 à 50 minutes environ, de préférence 5 à 30 minutes environ, les fibres kératiniques sont rincées, avantageusement lavées au shampooing, rincées à nouveau puis séchées ou laissées sécher.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11.

Il peut être ajusté à la valeur désirée au moyen d'agents acidifiants ou alcalinisants habituellement utilisés en teinture des fibres kératiniques et tels que définis précédemment.

La composition oxydante peut également renfermer divers adjuvants utilisés classiquement dans les compositions pour la teinture des cheveux et tels que définis précédemment.

La composition prête à l'emploi qui est finalement appliquée sur les fibres kératiniques, et qui correspond à la composition tinctoriale mélangée à la composition comprenant l'agent oxydant, peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme une composition tinctoriale éclaircissante contenant le colorant cationique mixte tel que défini ci-dessus et un deuxième compartiment renferme un agent oxydant.

Ce dispositif peut être équipé d'un moyen permettant de délivrer sur les cheveux le mélange souhaité, tel que les dispositifs décrits dans le brevet FR-2 586 913.

Les exemples ci-dessous illustrent la présente invention sans toutefois en limiter la portée.

### EXEMPLES

### EXEMPLE 1

### 1,A - Synthèse du dichlorhydrate de N-Méthyl-N-(2-méthylamino-éthyl)-benzène-1,4-diamine

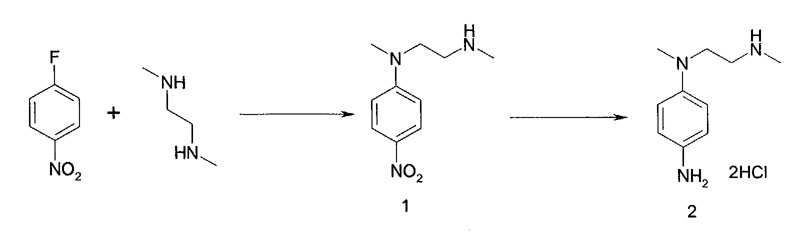

### Etape 1 : synthèse de la N,N"-diméthyl-N-(4-nitrophényl)éthane-1,2-diamine 1

5 g de 4-fluoronitrobenzène (35 mmoles) sont dissous dans 50 ml de THF. 2 équivalents de N,N-diméthylènediamine sont ajoutés goutte à goutte. Le milieu réactionnel est porté au reflux du THF pendant 20 heures. Le mélange est ensuite versé sur de la glace pilée, un précipité se forme. Ce dernier est filtré puis lavé à l'eau, puis séché.

### Etape 2 : synthèse du dichlorhydrate de N-Méthyl-N-(2-méthylamino-éthyl)-benzène-1,4-diamine 2

Dans un hydrogénateur de 1L, le composé nitré 1 est solubilisé dans 500ml d'éthanol. 10% de palladium sur charbon (50% humide) sont ajoutés et l'hydrogénateur est purgé à l'azote puis chargé en hydrogène. Après 1 h30 de réaction, les sels de palladium sont filtrés. 20 ml d'éthanol chlorhydrique 3M puis 300 ml d'ether isopropylique sont ajoutés. Le précipité obtenu est filtré et recristallisé dans l'éthanol chlorhydrique. Après filtration puis séchage, le produit est obtenu.
RMN ¹H (ppm) : 2,75 (s, 3H) ; 2,98 (s, 3H) ; 3,29 (t, 2H) ; 3,69 (t, 2H) ; 6,95 (m, 2H) ; 7,29 (m, 2H).
Masse : M = 179

| Analyse élémentaire : | | | | |
|---|---|---|---|---|
| Théorique | C : 47.63 | H :7.59 | N :16.66 | CI :27.67 |
| Mesuré | C : 47.04 | H :7.69 | N :15.75 | Cl :27.67 |

### 1-B - Exemple de teinture

On a préparé la composition tinctoriale suivante (teneurs en grammes) :

| **Constituant** | **g%** |
|---|---|
| Dichlorure de N-Methyl-N-(2-methylamino-ethyl)-benzene-1,4-diamine *(6 10*^{*-3*} *mole)* | 1,5 |
| Dichlorhydrate de 2,4-diamino-phénoxyéthanol (Coupleur *(6 10*^{*-3*} *mole)*) | 1,45 |
| Alcool oléïque polyglycérolé à 2 moles de glycérol | 4 |
| Alcool oléïque polyglycérolé à 4 moles de glycérol | 5,69 MA |
| Acide oléïque | 3 |
| Aminel oléïque à 2 moles d'oxyde d'éthylène vendue sous la dénomination commerciale ETHOMEEN 012 par la société Akzo | 7 |
| Laurylamino succinamate de diéthylaminopropyle , sel de sodium à 55% de M.A. | 3,0 MA |
| Alcool oléïque | 5 |
| Diethanolamide d'acide oléïque | 12 |
| Alcool éthylique | 7 |
| Propylène glycol | 3,5 |
| Dipropylèneglycol | 0,5 |
| Monométhylether de propylèneglycol | 9 |
| Antioxydant /séquestrant | qs |
| Acétate d'ammonium | 0,8 |
| Métabisulfite de sodium en solution aqueuse à 35% | 0,45 |
| Ammoniaque à 20 % | 10 |
| Eau déminéralisée q.s.p. | 100 g |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

La nuance obtenue sur mèches est un bleu puissant

### EXEMPLE 2

### 2.A - Synthèse du N-méthyl N-(6-méthylamino-hexyl)-benzène 1,4-diamine (4)

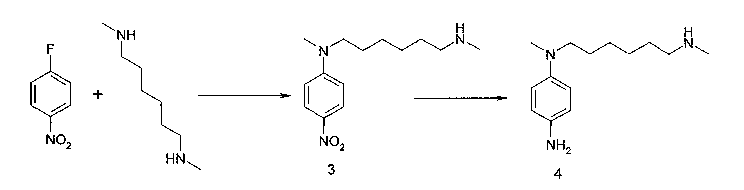

2 g de 4-fluoronitrobenzène (14 mmoles) sont dissous dans 8 ml de N-méthylpyrrolidone. Un équivalent de N,N'-Diméthyl-hexane-1,6-diamine (1,86 g) et 2,90 g de carbonate de potassium sont ajoutés. Le milieu réactionnel est chauffé à 80°C pendant 2 heures. Le mélange est ensuite versé sur de la glace pilée, un précipité se forme. Ce dernier est filtré puis lavé à l'eau, puis séché. Le composé nitré 3 ainsi obtenu est ensuite réduit au zinc.
Masse : LC/MS ESI + : m/z = 236 (M + 1), 259 (M + 1 + 23).

### 2.B - EXEMPLES DE TEINTURE EN MILIEU ACIDE

On a préparé les compositions tinctoriales suivantes :

| **Exemples** | **2.1** | **2.2** | **2.3** | **2.4** |
|---|---|---|---|---|
| N-Methyl-N-(6-methylamino-hexyl)benzene-1,4-diamine | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)ethanol,chlorhydrate | | | 10⁻³ mole | |
| 3-Amino-2-chloro-6-methylphenol,chlorhydrate | | | | 10⁻³ mole |
| Support de teinture (1) | (*) | (*) | (*) | (*) |
| Eau déminéralisée q.s.p. | 100g | 100g | 100g | 100g |

| | | | | |
|---|---|---|---|---|
| (*) Support de teinture (1) pH 7 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine- | 0,48 g M.A |
| pentaacétique en solution aqueuse à 40% | |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rincées, lavées avec un shampooing standard, rinçées à nouveau puis séchées.
Les résultats de teinture suivants ont été obtenus.

| **Exemples** | **2.1** | **2.2** | **2.3** | **2.4** |
|---|---|---|---|---|
| **Nuance observée** | gris | brun | gris vert-bleu intense | bleu |

### 2.C - EXEMPLES DE TEINTURE EN MILIEU ALCALIN

| **Exemple** | **2.5** | **2.6** | **2.7** | **2.8** | **2.9** |
|---|---|---|---|---|---|
| N-Methyl-N-(6methylamino-hexyl)benzene-1,4-diamine | 10⁻³ mole | 10⁻³ mole | 10⁻³ mole | 10⁻³ mole | 10⁻³ mole |
| 5-Amino-2-methyl-phenol | 10⁻³ mole | | | | |
| 1H-Indol-6-ol | | 10⁻³ mole | | | |
| 3,6-Dimethyl-1 Hpyrazolo[5,1-c][1,2 ,4]triazole | | | 10⁻³ mole | | |
| 2-(2,4-Diaminophenoxy)ethanol,chlorhydrate | | | | 10⁻³ mole | |
| 3-Am ino-2-chloro-6-methylphenol,chlorhydrate | | | | | 10⁻³ mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) Support de teinture (2) pH 9,5 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique | 0,48 g M.A |
| en solution aqueuse à 40% | |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **2.5** | **2.6** | **2.7** | **2.8** | **2.9** |
|---|---|---|---|---|---|
| **Nuance observée** | Violet-bleu intense | orangé | Violet-rouge chromatique | Vert-bleu | bleu |

### EXEMPLE 3

### 3.A - Synthèse de N-(4-amino-3-methylphenyl)-N-methyl-N-[6-(methylamino)hexyl]amine (6)

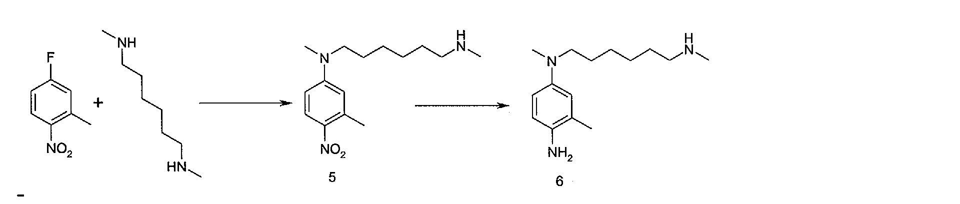

2 g de 4-Fluoro-2-methyl-1-nitro-benzene (14 mmoles) sont dissous dans 8 ml de N-Méthyl pyrrolydinone. Un équivalent de N,N'-Dimethyl-hexane-1,6-diamine (1,86 g) et 2,90 g de carbonate de potassium sont ajoutés. Le milieu réactionnel est chauffé à 80°C pendant 2 heures. Le mélange est ensuite versé sur de la glace pilée, un précipité se forme. Ce dernier est filtré puis lavé à l'eau, puis séché. Le composé nitré 5 ainsi obtenu est ensuite réduit au zinc.
Masse : LC/MS ESI + : m/z = 250 (M + 1), 273 (M + 1 + 23).

### 3.B - EXEMPLES DE TEINTURE EN MILIEU ACIDE

On a préparé les compositions tinctoriales suivantes :

| **Exemple** | **3.1** | **3.2** | **3.3** | **3.4** | **3.5** | **3.6** | **3.7** |
|---|---|---|---|---|---|---|---|
| N-(4-amino-3-methylphenyl)-N-methyl-N-[6-(methylamino)hexyl]amine | 10⁻³ mole | 10⁻³ mole | 10⁻³ mole | 10⁻³ mole | 10⁻³ mole | 10⁻³ mole | 10⁻³ mole |
| Benzene-1,3-diol | 10⁻³ mole | | | | | | |
| 5-Amino-2-methyl-phenol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Dimethyl-1H-pyrazolo[5,1-c][1,2 ,4]triazole | | | | | 10⁻³ mole | | |
| 2-(2,4-Diamino-phenoxy)ethanol,chlorhydrate | | | | | | 10⁻³ mole | |
| 3-Amino-2-chloro-6-methylphenol,chlorhydrate | | | | | | | 10⁻³ mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rincées, lavées avec un shampooing standard, rincées à nouveau puis séchées.
Les résultats de teinture suivants ont été obtenus.

| **Exemples** | **3.1** | **3.2** | **3.3** | **3.4** | **3.5** | **3.6** | **3.7** |
|---|---|---|---|---|---|---|---|
| **Nuance observée** | jaune | gris violet intense | gris intense | brun | orangé | vert-bleu intense | bleu intense |

### 3.C - EXEMPLES DE TEINTURE EN MILIEU ALCALIN

| **Exemples** | **3.8** | **3.9** | **3.10** | **3.11** | **3.12** | **3.13** | **3.14** |
|---|---|---|---|---|---|---|---|
| N-(4-amino-3-methylphenyl)-N-methyl-N-[6-(methylamino)hexyl]amine | 10⁻³ mole | 10⁻³ mole | 10⁻³ mole | 10⁻³ mole | 10⁻³ mole | 10⁻³ mole | 10⁻³ mole |
| Benzene-1,3-diol | 10⁻³ mole | | | | | | |
| 5-Amino-2-methyl-phenol | | 10⁻³ mole | | | | | |
| 1H-Indol-6-ol | | | 10⁻³mole | | | | |
| 2-Amino-pyridin-3-ol | | | | 10⁻³mole | | | |
| 3,6-Dimethyl-1H-pyrazolo[5,1-c][1,2 ,4]triazole | | | | | 10⁻³ mole | | |
| 2-(2,4-Diamino-phenoxy)ethanol,chlorhydrate | | | | | | 10⁻³ mole | |
| 3-Amino-2-chloro-6-methyl-phenol,chlorhydrate | | | | | | | 10⁻³ mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rincées, lavées avec un shampooing standard, rinçées à nouveau puis séchées.
Les résultats de teinture suivants ont été obtenus.

| **Exemples** | **3.8** | **3.9** | **3.10** | **3.11** | **3.12** | **3.13** | **3.14** |
|---|---|---|---|---|---|---|---|
| **Nuance observée** | brun jaune | violet intense | rouge | brun rouge | violet-rouge chromatique intense | bleu intense | violet-bleu intense |

## Revendications

1. Composé de formule suivante (I) 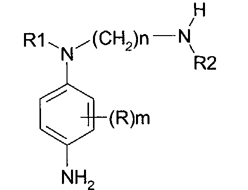 ainsi que ses sels d'addition,
dans laquelle :
• R représente un radical alkyle, linéaire ou ramifié, en C₁-C₄ ; un radical alcoxy, linéaire ou ramifié, en C₁-C₄ ;
• m représente un entier compris entre 0 et 4 ;
• R₁ représente un radical alkyle, linéaire ou ramifié, en C₁-C₄ ; un radical hydroxyalkyle, linéaire ou ramifié, en C₂-C₄ ; un radical alcoxy(C₁-C₄) alkyle en C₂-C₄, linéaire ou ramifié ;
• R₂ représente un atome d'hydrogène ; un radical alkyle, linéaire ou ramifié, en C₁-C₄ ; un radical hydroxyalkyle, linéaire ou ramifié, en C₂-C₄ ; un radical alcoxy(C₁-C₄) alkyle en C₂-C₄, linéaire ou ramifié.
• n représente un entier compris entre 2 et 6.

2. Composé selon la revendication précédente, **caractérisé en ce que** le composé de formule (I) est choisi parmi les composés suivants : 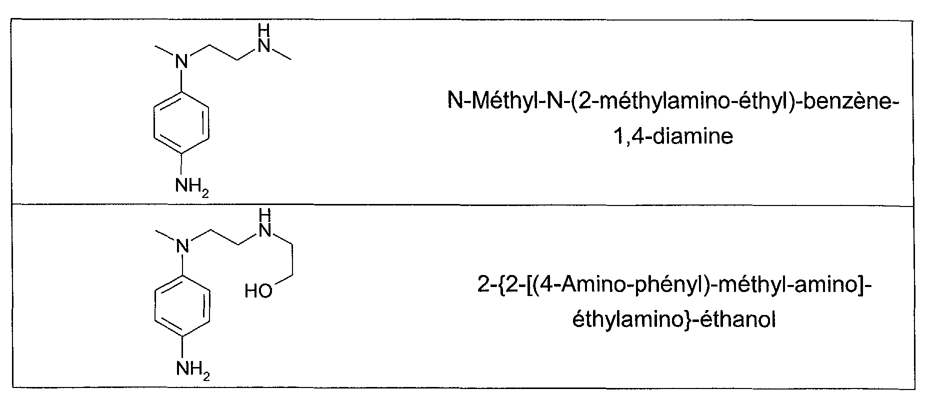 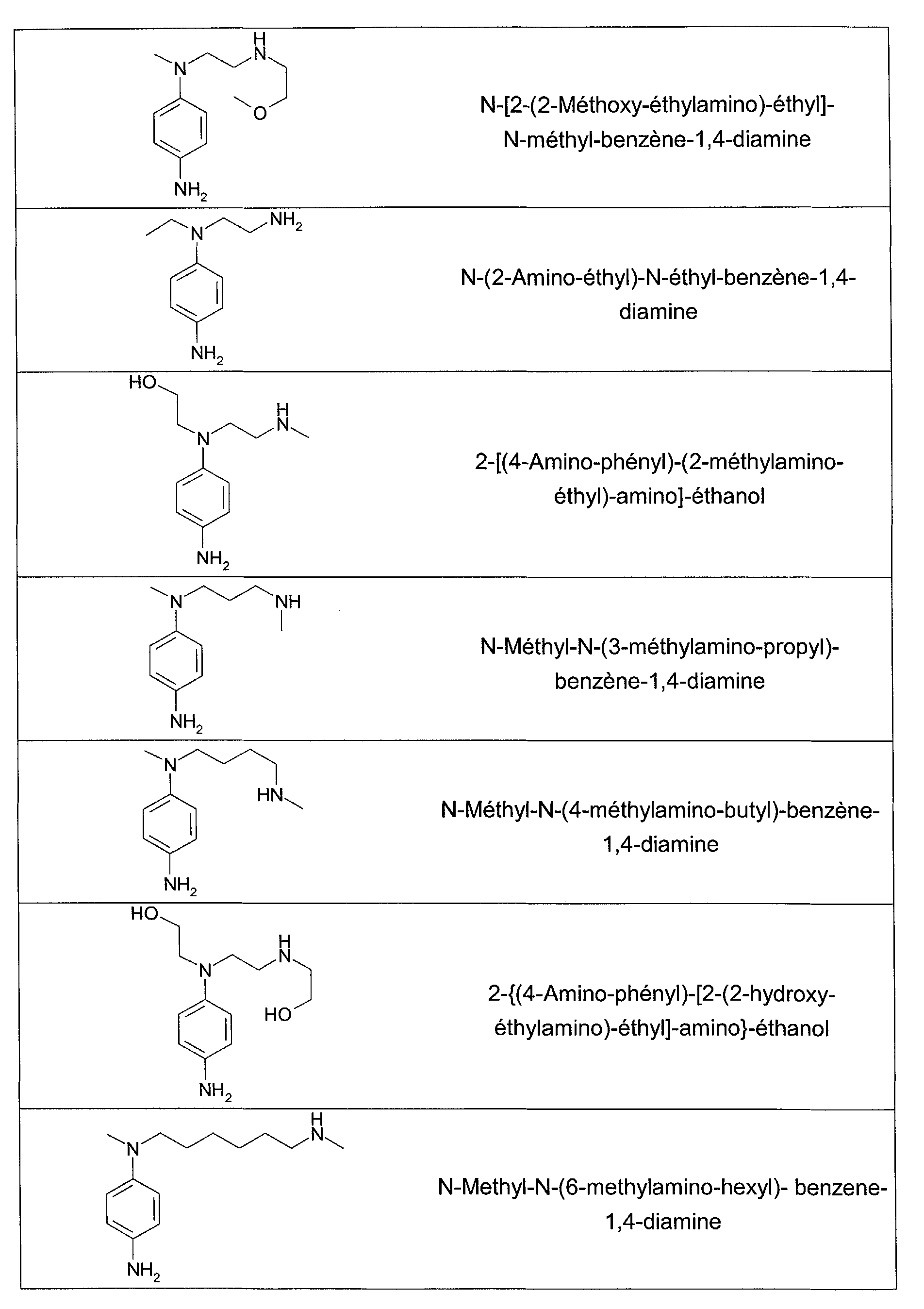 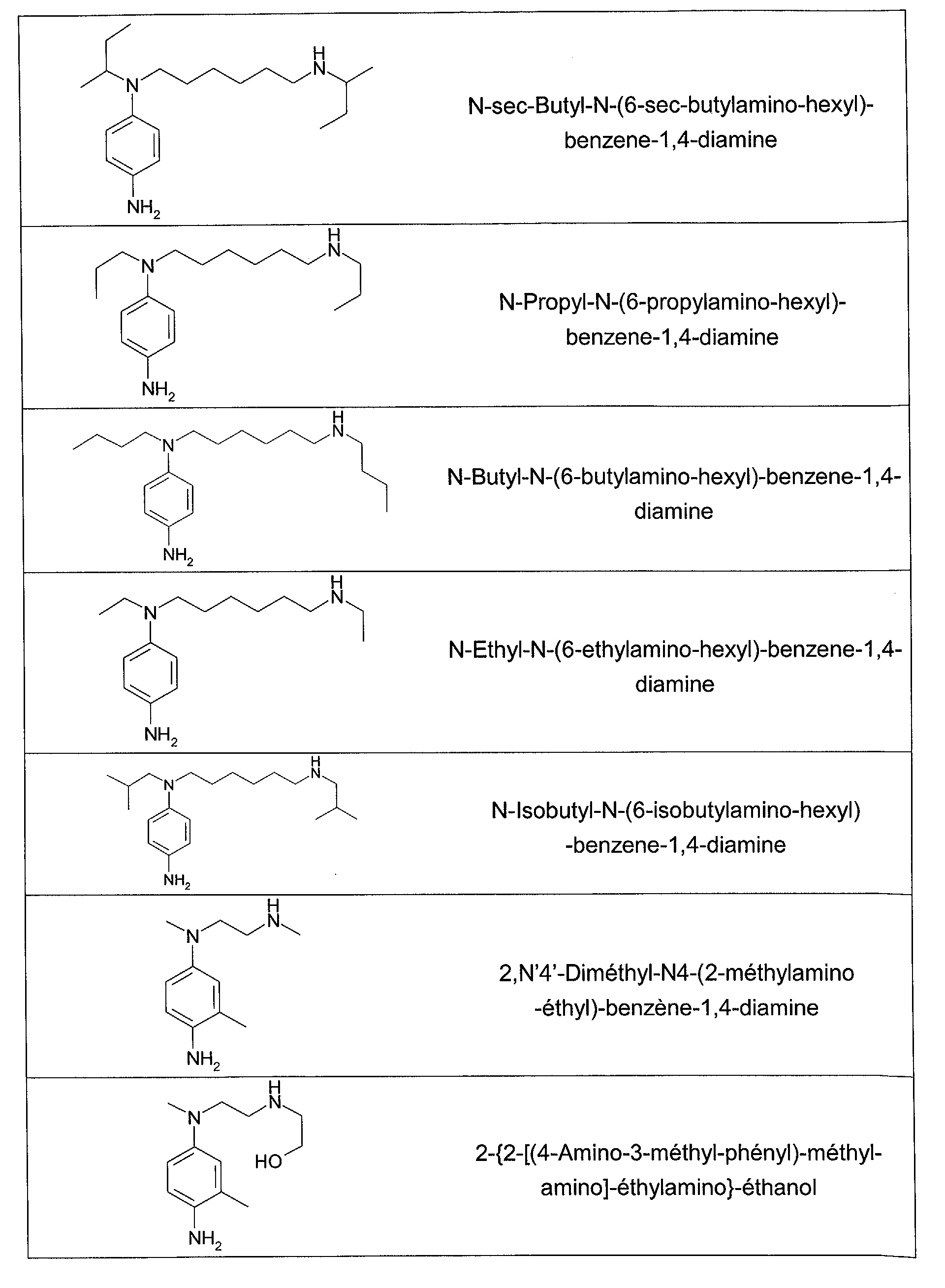 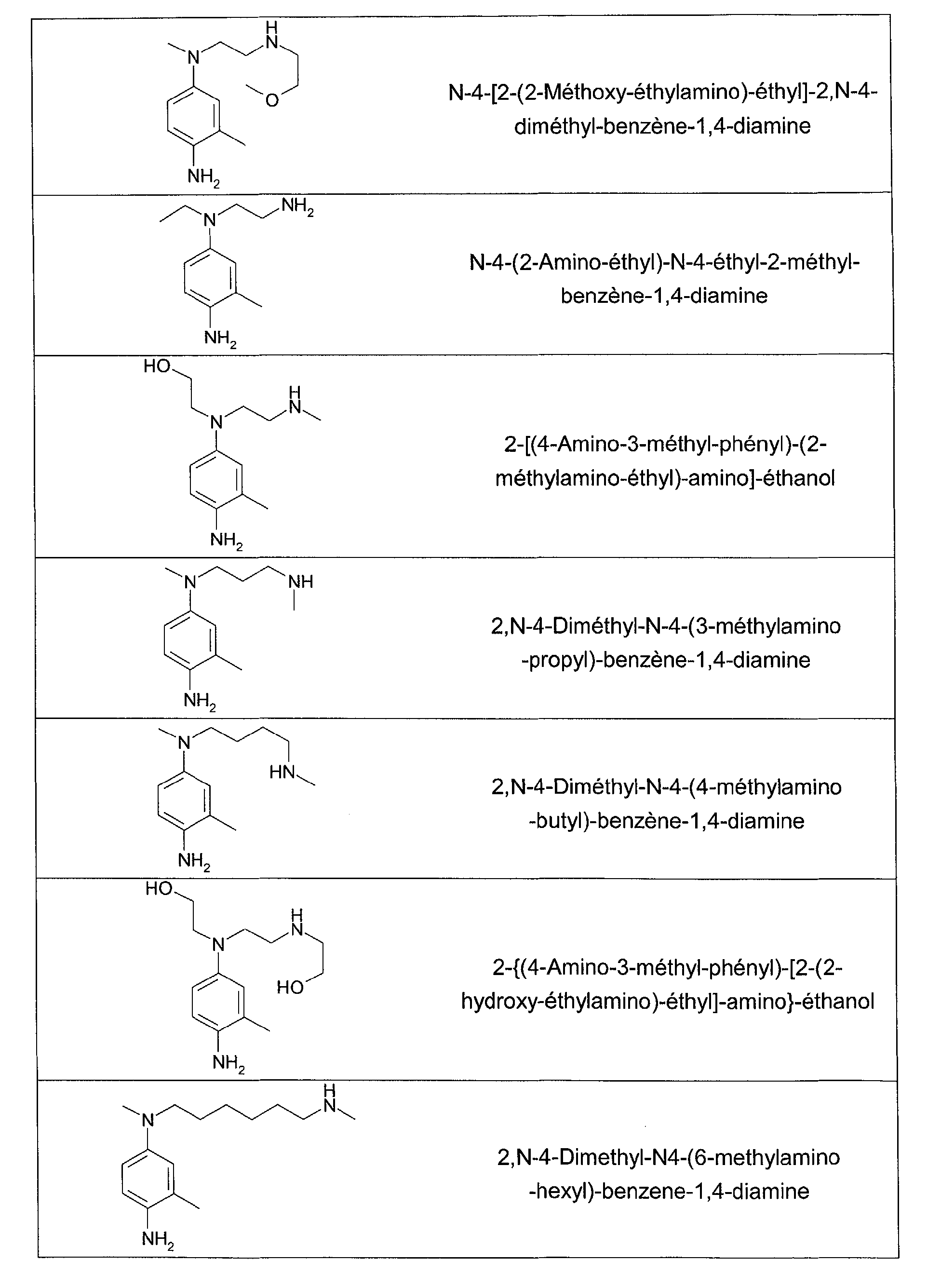 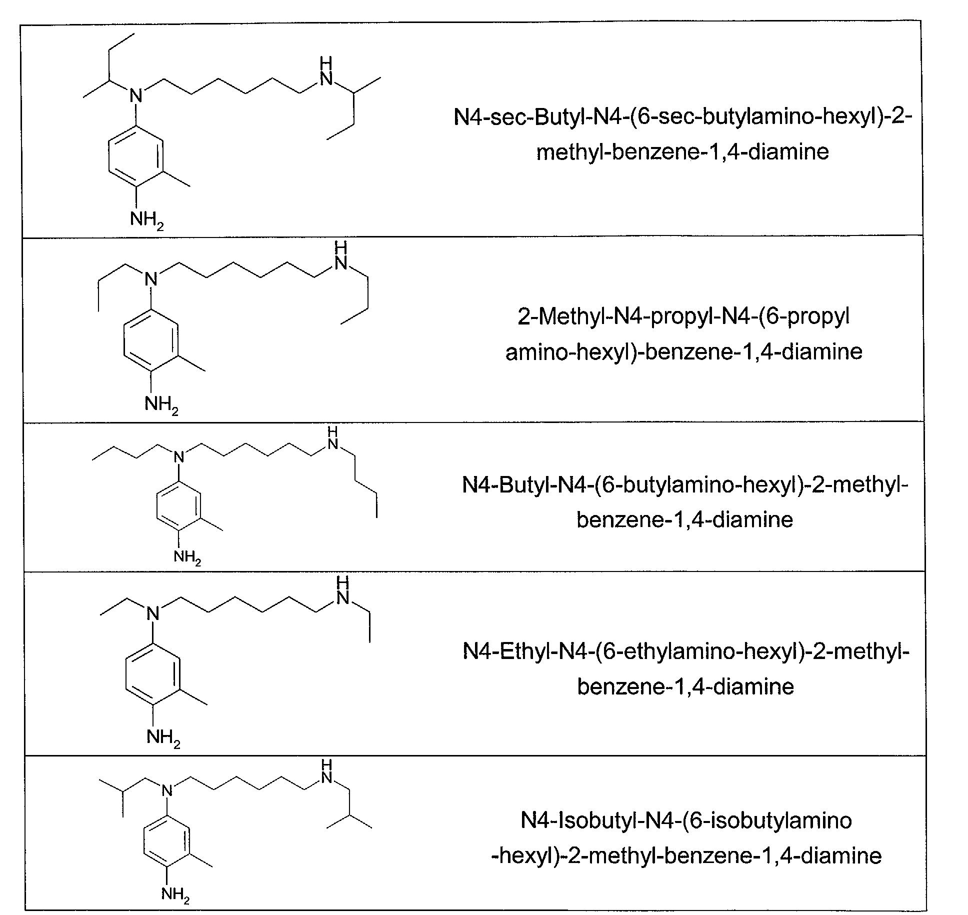 ainsi que leurs sels d'addition.

3. Composition tinctoriale comprenant, dans un milieu approprié pour la teinture des fibres kératiniques, en particulier humaines, au moins un composé de formule (I) tel que défini selon l'une des revendications 1 ou 2.

4. Composition selon la revendication précédente, **caractérisée en ce que** la teneur en composé de formule (I) représente de 0,001 à 10 % en poids par rapport au poids de la composition tinctoriale, de préférence de 0,005 à 6 % en poids par rapport au poids de la composition tinctoriale.

5. Composition selon l'une quelconque des revendications 3 ou 4, **caractérisée en ce qu'**elle comprend au moins une base d'oxydation additionnelle éventuellement associée à au moins un coupleur.

6. Composition selon la revendication précédente, **caractérisée en ce que** la base d'oxydation additionnelle est choisie parmi les paraphénylènediamines différente du composé de formule (I), les bis-phénylalkylènediamines, les para-aminophénols, les ortho-aminophénols, les bases hétérocycliques et leurs sels d'addition.

7. Composition selon la revendication précédente, **caractérisée en ce que** la teneur en base d'oxydation additionnelle est comprise entre 0,001 à 10 % en poids par rapport au poids de la composition tinctoriale.

8. Composition selon l'une des revendications 5 à 7, **caractérisée en ce que** le coupleur, de préférence choisi parmi les métaphénylènediamines, les méta-aminophénols, les métadiphénols, les coupleurs naphtaléniques, les coupleurs hétérocycliques et leurs sels d'addition.

9. Composition selon la revendication précédente, **caractérisée en ce que** la teneur en coupleur est comprise entre 0,001 et 10 % en poids par rapport au poids de la composition tinctoriale.

10. Composition selon l'une quelconque des revendications 3 à 9, **caractérisée en ce qu'**elle comprend au moins un colorant direct, neutre, anionique ou cationique.

11. Composition selon la revendication précédente, **caractérisée en ce que** le colorant direct est choisi parmi les colorants de type les colorants benzéniques nitrés, les colorants azoïques, azométhiniques, aziniques, méthiniques, tétraazapenthaméthiniques, quinoniques, anthraquinoniques, naphtoquinoniques, benzoquinoniques, phénotiaziniques, indigoïdes, indoaminiques, xanthéniques, phénanthridiniques, phtalocyanines, ceux dérivés du triarylméthane, les colorants naturels, seuls ou en mélanges.

12. Composition selon la revendication précédente, **caractérisée en ce que** la teneur en colorant direct représente de 0,001 à 20 % en poids par rapport au poids de la composition tinctoriale, de préférence de 0,01 à 10 % en poids par rapport au poids de la composition tinctoriale.

13. Composition selon l'une quelconque des revendications 3 à 12, **caractérisée en ce qu'**elle comprend au moins un agent oxydant.

14. Composition selon la revendication précédente, **caractérisée en ce que** l'agent oxydant est chois parmi le peroxyde d'hydrogène, le peroxyde d'urée, les bromates de métaux alcalins, les persels tels que les perborates et persulfates, les enzymes telles que les peroxydases et les oxydo-réductases à deux ou à quatre électrons, et de préférence le peroxyde d'hydrogène.

15. Procédé de teinture d'oxydation des fibres kératiniques, notamment humaines, **caractérisé en ce qu'**on applique sur les fibres sèches ou humides, une composition tinctoriale telle que définie dans l'une quelconque des revendications 3 à 14 pendant un temps suffisant pour obtenir la coloration désirée.

16. Procédé selon la revendication précédente, **caractérisé en ce que** l'agent oxydant est contenu dans une composition différente de la composition selon l'une quelconque des revendications 1 à 12.

17. Dispositif à plusieurs compartiments dans lequel un premier compartiment contient une composition tinctoriale selon l'une des revendications 1 à 12 et un deuxième compartiment contenant un agent oxydant.
